# EUROPEAN PATENT SPECIFICATION

(11) **EP 1 759 651 B1**
(45) Date of publication and mention of the grant of the patent: **26.08.2009**
(21) Application number: 06018206.0
(22) Date of filing: 31.08.2006
(51) Int. Cl.: A61B 18/18

(54) **Return electrode pad with conductive element grid**
Neutralelektroden-Pad mit leitendem Gitterelement
Electrode de retour plate avec grille d'éléments conducteurs

(30) Priority: 01.09.2005 US 218110
(43) Date of publication of application: 07.03.2007
(73) Proprietor: Covidien AG, 8212 Neuhausen am Rheinfall (CH)
(72) Inventor: Eggleston, Jeffrey L., Broomfield Colorado 80020 (US)
(74) Representative: HOFFMANN EITLE

(56) References cited:
- EP-A- 1 051 949
- WO-A-00/53113
- WO-A-98/53751
- US-A- 4 343 308

## Description

### BACKGROUND

### Technical Field

The present disclosure is directed to an electrosurgical apparatus and, is particularly directed to a patient return electrode pad containing grids and an apparatus for performing monopolar surgery using the same.

### Background

During electrosurgery, a source or active electrode delivers energy, such as radio frequency energy, from an electrosurgical generator to a patient. A return electrode carries the current back to the electrosurgical generator. In monopolar electrosurgery, the source electrode is typically a hand-held instrument placed by the surgeon at the surgical site and the high current density flow at this electrode creates the desired surgical effect of cutting and/or coagulating tissue. The patient return electrode is placed at a remote site from the source electrode and is typically in the form of a pad adhesively adhered to the patient.

The return electrode typically has a relatively large patient contact surface area to minimize heating at that site because the smaller the surface area, the greater the current density and the greater the intensity of the heat. That is, the area of the return electrode that is adhered to the patient is generally important because it is the current density of the electrical signal that heats the tissue. A larger surface contact area is desirable to reduce heat intensity. The size of return electrodes is based on assumptions of the maximum current seen in surgery and the duty cycle (e.g., the percentage of time the generator is on) during the procedure. The first types of return electrodes were in the form of large metal plates covered with conductive jelly. Later, adhesive electrodes were developed with a single metal foil covered with conductive jelly or conductive adhesive. However, one problem with these adhesive electrodes was that if a portion peeled from the patient, the contact area of the electrode with the patient decreased, thereby increasing the current density at the adhered portion and, in turn, increasing the heat applied to the tissue. This risked burning the patient in the area under the adhered portion of the return electrode if the tissue was heated beyond the point where circulation of blood could cool the skin.

To address this problem, split return electrodes and hardware circuits, generically called Return Electrode Contact Quality Monitors (RECQMs), were developed. These split electrodes consist of two separate conductive foils arranged as two halves of a single return electrode. The hardware circuit uses an AC signal between the two electrode halves to measure the impedance therebetween. This impedance measurement is indicative of how well the return electrode is adhered to the patient since the impedance between the two halves is directly related to the area of patient contact. That is, if the electrode begins to peel from the patient, the impedance increases since the contact area of the electrode decreases. Current RECQMs are designed to sense this change in impedance so that when the percentage increase in impedance exceeds a predetermined value or the measured impedance exceeds a threshold level, the electrosurgical generator is shut down to reduce the chances of burning the patient.

As new surgical procedures continue to be developed that utilize higher current and higher duty cycles, increased heating of tissue under the return electrode may occur. It would therefore be advantageous to design a return electrode pad which has the ability of reducing the likelihood of patient burns, while still being able to dissipate an increased amount of heat.
US-A-4 343 308 discloses an electrosurgical grounding pad with a temperature sensor. As the temperature of the grounding pad approaches a potentially dangerous level, an alarm indicates to operating room personnel the possible burn condition.
EP 1 051 949 discloses an electrosurgical return electrode monitor. The preamble of claim 1 is based on this document.

### SUMMARY

The present disclosure provides an electrosurgical return electrode for use in monopolar surgery. The return electrode comprises a conductive pad including a plurality of conductive elements. The return electrode further includes a plurality of temperature sensors which are each operatively engaged with a respective one of the plurality of conductive elements on a top surface of the conductive pad and which measure the temperature of a portion of a patient in contact with the respective conductive element.

The present disclosure may also include a connection device which selectively enables the transfer of radio frequency current from an active electrode to at least one of the plurality of conductive elements. In operation, the connection device may be connected, disconnected, activated, deactivated and/or adjusted to a conductive element when the temperature of the patient in contact with the respective conductive element reaches a predetermined level. Specifically, if the temperature of a portion of the patient is too high, the conductive element contacting the patient at that location may be disconnected from the connection device. If the temperature of a portion of the patient in contact with a conductive element is cool enough, the conductive element in that location can be connected (or reconnected) to the connection device.

It is envisioned for the plurality of conductive elements to form a grid. Additionally, each of the conductive elements may be approximately the same size. Alternatively, certain conductive elements may be a different size from the rest. For example, the conductive elements around the perimeter of the conductive pad may be relatively smaller than the remainder of the conductive elements.

In an embodiment, an adhesive portion is included on the electrosurgical return electrode which facilitates the adhesion between at least a portion of the conductive pad and a patient. This adhesive portion may be capable of conducting electricity.

In a particularly useful embodiment, the connection device is connectable to an electrosurgical generator and to each of the plurality of the conductive elements.

It is envisioned for each of the temperature sensors to be able to measure the temperature of a patient's skin in contact therewith and/or in contact with the corresponding conductive element.

The connection device may be located on the conductive pad, on an electrosurgical generator, or at a location between the conductive pad and the electrosurgical generator.

It is envisioned for the electrosurgical return electrode to be entirely disposable, partially disposable, or entirely re-usable. It is further envisioned for some portions of the electrosurgical return electrode to be disposable and for some portions to be re-usable. For example, the conductive elements may be re-usable, while an adhesive may be disposable.

The present disclosure also provides an electrosurgical apparatus for performing electrosurgery on a patient according to claim 12.

For a better understanding of the present disclosure and to show how it may be carried into effect, reference will now be made by way of example to the accompanying drawings.

### BRIEF DESCRIPTION OF THE DRAWINGS

The above and other aspects, features, and advantages of the present disclosure will become more apparent in light of the following detailed description when taken in conjunction with the accompanying drawings in which:

FIG. 1 is a schematic illustration of a monopolar electrosurgical system;

FIG. 2 is a plan view of an electrosurgical return electrode according to an embodiment of the present disclosure, illustrating a conductive pad having a grid of conductive elements of substantially equal sizes;

FIG. 3 is a plan view of an electrosurgical return electrode according to an embodiment of the present disclosure, illustrating a conductive pad having a grid of conductive elements of various sizes; and

FIG. 4 is an enlarged schematic cross-sectional view of a portion of the return electrodes of FIGS. 1-3.

### DETAILED DESCRIPTION

Embodiments of the presently disclosed temperature regulating patient return electrode of using the same will be described herein below with reference to the accompanying drawing figures wherein like reference numerals identify similar or identical elements. In the following description, well-known functions or constructions are not described in detail to avoid obscuring the disclosure in unnecessary detail.

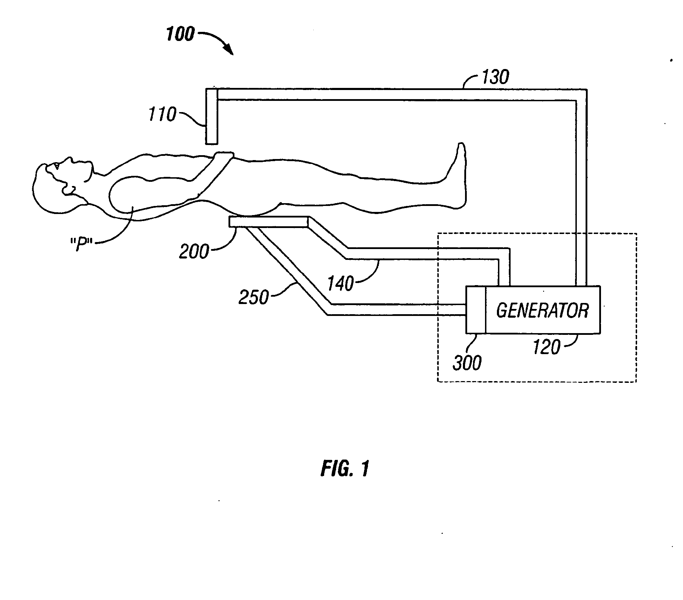

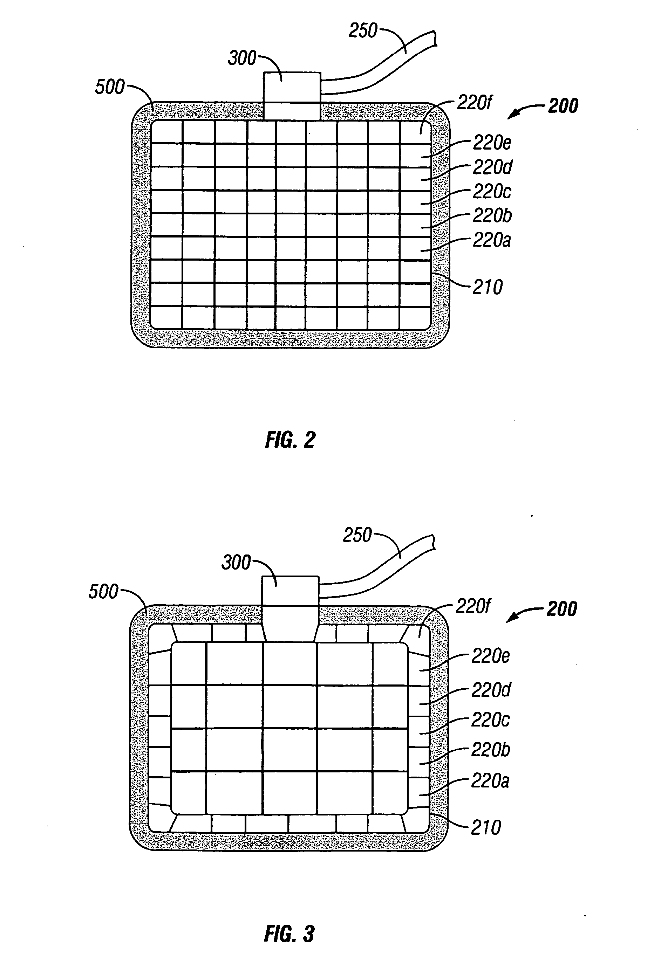

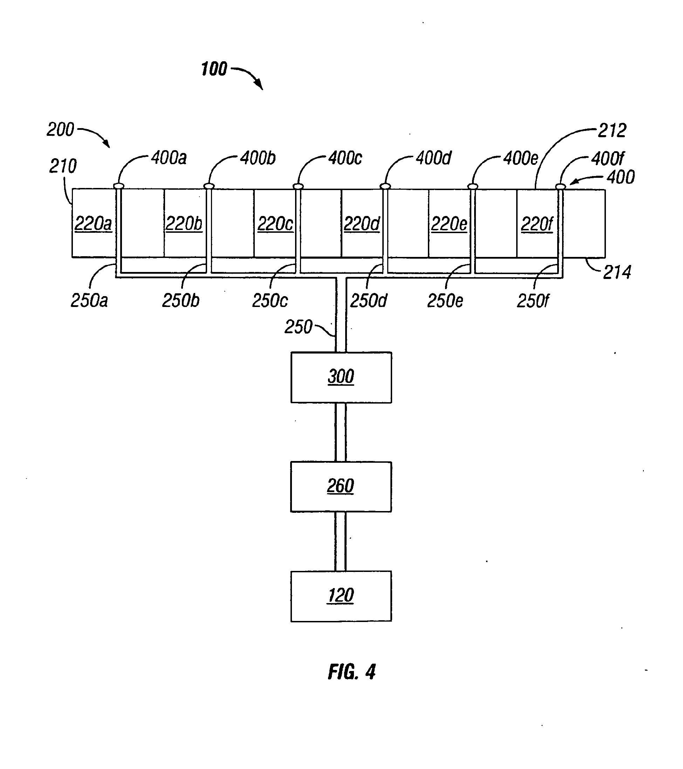

Referring initially to FIG. 1, a schematic illustration of a monopolar electrosurgical system 100 is shown. The electrosurgical system generally includes a return electrode 200, a connection device 300 for connecting the return electrode 200 to a generator 120, and a plurality of temperature sensors 400 disposed on or operatively associated with the return electrode 200 (FIG. 4). In FIG. 1, the return electrode 200 is illustrated placed under a patient "P." The plurality of temperature sensors 400 are in operative engagement with the return electrode 200 and operatively connect to the connection device 300 via a second cable 250. The connection device 300 may be operatively connected to the generator 120 (FIG. 1), may be operatively connected to the return electrode 200 (FIGS. 2 and 3), or may be disposed between the return electrode 200 and a generator 120 (FIG. 4).

A surgical instrument (e.g., an active electrode) for treating tissue at the surgical site is designated by reference number 110. Electrosurgical energy is supplied to the surgical instrument 110 by the generator 120 via a first cable 130 to cut, coagulate, blend, etc. tissue. The return electrode 200 returns the excess energy delivered by the surgical instrument 110 to the patient "P" back to the generator 120 via a wire 140. It is envisioned for the wire 140 to be incorporated into the second cable 250.

FIGS. 2, 3 and 4 illustrate various embodiments of the return electrode 200 for use in monopolar electrosurgery. Generally, the return electrode 200 is a conductive pad 210 having a top surface 212 (FIG. 4) and a bottom surface 214 (FIG. 4). The return electrode 200 is designed and configured to receive current during monopolar electrosurgery. While the figures depict the return electrode 200 in a general rectangular shape, it is within the scope of the disclosure for the return electrode 200 to have any regular or irregular shape.

As illustrated in FIGS. 2, 3 and 4, the conductive pad 210 is comprised of a plurality of conductive elements (only conductive elements 220a - 220f are labeled for clarity) arranged in a regular or irregular array. Each of the plurality of conductive elements 220 may be equally-sized or differently-sized and may form a grid/array or be disposed in any other grid-like arrangement on the conductive pad 210. It is also envisioned and within the scope of the present disclosure for the plurality of conductive elements 220 to be arranged in a spiral or radial orientation (not shown) on the conductive pad 210. While the figures depict the conductive elements 220 in a generally rectangular shape, it is within the scope of the present disclosure for the conductive elements 220 to have any regular or irregular shape.

As illustrated in FIG. 4, the plurality of temperature sensors 400 include individual temperature sensors (illustrated as 400a-400f , corresponding to conductive elements 220a - 220f, respectively), which are able to measure the temperature of a patient's skin in contact therewith. The plurality of temperature sensors 400 are operatively connected to the plurality of conductive elements 220 on the top surface 212 of the conductive pad 210. In such an arrangement, one of the plurality of temperature sensors 400 is operatively connected to one of the plurality of conductive elements 220. For example, individual temperature sensor 400a may be operatively connected to conductive element 220a. Each of the plurality of temperature sensors 400 is connected to the connection device 300 via a respective second cable 250. For example, temperature sensor 400a may be connected to the connection device 300 via second cable 250a. In the interest of clarity, each of the second cables 250 connected to each of the temperature sensors 400 is not illustrated in FIGS. 2 and 3.

Generally, the area of the return electrode 200 that is in contact with the patient "P" affects the current density of a signal that heats the patient "P." The smaller the contact area the return electrode 200 has with the patient "P," the greater the current density and the greater and more concentrated the heating of tissue is. Conversely, the greater the contact area of the return electrode 200, the smaller the current density and the less heating of the tissue. Further, the greater the heating of the tissue, the greater the probability of burning the tissue. It is therefore important to either ensure a relative high amount of contact area between the return electrode 200 and the patient "P," or otherwise maintain a relatively low current density on the return electrode 200.

While there are various methods of maintaining a relatively low current density (including, inter alia, the use of electrosurgical return electrode monitors (REMs), such as the one described in commonly-owned U.S. Patent No. 6,565,559), the present disclosure ensures the return electrode 200 maintains a low current density by monitoring the temperature of each of the plurality of conductive elements 220 of the return electrode 200.

Each temperature sensor 400 of the present disclosure has the ability to measure the temperature of the patient "P" that is in contact therewith. Further, each conductive element 220 of the present disclosure may be connected and/or disconnected to the connection device 300 or may be activated and/or deactivated as needed, or may be adjusted as needed. When the temperature of the patient "P" in contact with a particular conductive element 220 reaches a predetermined level, that conductive element 220 may either be connected, disconnected, activated, deactivated or adjusted as needed. For example, if a conductive element (e.g., 220a ) along the perimeter of the conductive pad 210 becomes relatively hot, that conductive element 220a may be disconnected from the connection device 300, deactivated or adjusted to receive a lower amount of energy. In this example, the conductive element 220a would not receive any more energy or receive a reduced amount of energy and the temperature in the area of the patient "P" contacting the conductive element 220a would consequently no longer rise. It is envisioned and within the scope of the present disclosure for the disconnection/re-connection, deactivation/reactivation of the conductive elements 220 to occur automatically as a result of an algorithm or the like provided in the electrosurgical generator 120.

It is also envisioned and within the scope of the present disclosure for a disconnected conductive element, e.g., 220a, to be reconnected to the connection device 300 when the temperature of the patient "P" in contact with the corresponding temperature sensor 400a falls to a relatively lower temperature (i.e., cools down). Utilizing these features, the temperature of the return electrode 200 can be relatively consistent throughout the entire surface thereof, thus reducing the possibility of "hot spots" and patient burns.

During electrosurgical use of the return electrode 200, portions of the perimeter of the return electrode 200 may become hot at a faster rate than the center of the return electrode 200. In such a situation, as seen in FIG. 3, it may be desirable to have the conductive elements 220 near the perimeter of the return electrode 200 be smaller than the remaining conductive elements 220. Monitoring the temperature of the patient "P" in contact with the smaller conductive elements 220 would allow greater control of the overall temperature of the portions of the patient "P" in contact with the return electrode 200. Thus, the return electrode 200, as a whole, would be able to receive a greater amount of current, as some new procedures necessitate.

To further limit the possibility of patient burns, it is envisioned for an adhesive layer 500 to be disposed on the return electrode 200, as illustrated in FIGS. 2 and 3. The adhesive layer 500 may be conductive and may be made from materials that include, but are not limited to, a polyhesive adhesive; a Z axis adhesive; or a water-insoluble, hydrophilic, pressure-sensitive adhesive and is desirably made of a polyhesive adhesive. Such materials are described in U.S. Patent Nos. 4,699,146 and 4,750,482. A function of the adhesive layer 500 is to ensure an optimal surface contact area between the return electrode 200 and the patient "P" and thus to limit the possibility of a patient burn.

It is envisioned for the return electrode 200 to be entirely disposable, entirely re-usable, or a combination thereof. In one embodiment, the conductive elements 220 are re-usable, while the adhesive layer 500 is disposable. Other combinations of disposable/reusable portions of the return electrode 200 are envisioned and within the scope of the present disclosure.

It is envisioned that a multiplexer 260 may be employed to control switching of the plurality of conductive elements 220, as illustrated in Fig. 4. For example, it is envisioned that the multiplexer 260 may be configured to regulate the current in any fashion by switching on and off various amounts of the plurality of conductive elements 220. While the multiplexer 260 is illustrated between the generator 120 and the connection device 300, other locations for the multiplexer 260 are envisioned and within the scope of the present disclosure.

There is also disclosed a method of performing monopolar electrosurgery. The method includes providing a return electrode 200 as described above; placing the return electrode 200 in contact with a patient "P"; generating electrosurgical energy via the generator 120; supplying the electrosurgical energy to the patient "P" via the active electrode 110; measuring the temperature of the portions of the patient "P" in contact with the plurality of conductive elements 220 via the plurality of temperature sensors 400; and monitoring the temperature of the portions of the patient "P" in contact with the plurality of conductive elements 220. Utilizing this method, a conductive element (e.g., 220a) may be disconnected or deactivated from the connection device 300 when the portion of the patient "P" in contact with the conductive element 220a reaches a predetermined temperature. Additionally, a conductive element (e.g., 220a) may be connected (or reconnected) to the connection device 300, or re-activated when the portion of the patient "P" in contact with that conductive element 220b falls to a predetermined temperature. As can be appreciated, this method can be utilized to maintain a relatively constant temperature where the return electrode 200 contacts the patient "P."

While several embodiments of the disclosure have been shown in the drawings, the above description should be construed merely as exemplifications of preferred embodiments. For example, it is envisioned for the return electrode 200 to be at least partially coated with a positive temperature coefficient (PTC) material to help distribute the heat across the return electrode 200.

## Claims

1. An electrosurgical return electrode pad (200) for use in monopolar surgery, comprising:
a conductive pad (210) including a plurality of conductive elements (220a-220f) and having a top surface (212) and a bottom surface (214), the conductive pad defining a perimeter; **characterised in that**:
the return electrode further comprises a plurality of temperature sensors, each temperature sensor being operatively engaged with a respective one of the plurality of conductive elements (220a-220f) on the top surface of the conductive pad, wherein each temperature sensor is configured to measure a temperature of a portion of a patient(P) in contact with the respective conductive element (220a-220f).

2. The electrosurgical return electrode (200) according to claim 1, further comprising a connection device (300) which selectively enables the transfer of radio frequency energy from an active electrode to at least one of the plurality of conductive elements (220a-220f), wherein the connection device (300) may be at least one of connected, disconnected, activated, deactivated and adjusted to a conductive element when the temperature of a corresponding portion of the patient (P) reaches a predetermined level.

3. The electrosurgical return electrode (200) according to any of the preceding claims, wherein the plurality of conductive elements (220a-220f) forms a grid.

4. The electrosurgical return electrode (200) according to any of the preceding claims, wherein each of the plurality of conductive elements (220a-220f) is approximately the same size.

5. The electrosurgical return electrode (200) according to any of claims 1 to 3, wherein at least one of the plurality of conductive elements (220a-220f) is a different size than the remainder of the conductive elements.

6. The electrosurgical return electrode (200) according to claim 5, wherein the conductive elements (220a-220f) along the perimeter of the conductive pad are relatively smaller than the remainder of the conductive elements.

7. The electrosurgical return electrode (200) according to any of the preceding claims, further comprising an adhesive portion (500) which adheres at least a portion of the conductive pad (210) to a patient (P).

8. The electrosurgical return electrode (200) according to claim 2 or any of claims 3 to 7 when dependent on claim 2, wherein the connection device (300) transfers radio frequency energy from an active electrode to each of the plurality of conductive elements (220a-220f).

9. The electrosurgical return electrode (200) according to any of the preceding claims, wherein each temperature sensor (400a-400f) is adapted to contact a patient (P).

10. The electrosurgical return electrode (200) according to claim 2 or any of claims 3 to 9 when dependent on claim 2, wherein the connection device (300) is i) disposed on the conductive pad (210) ii) disposed on an electrosurgical generator (120) or iii) disposed between the conductive pad (210) and an electrosurgical generator (120).

11. The electrosurgical return electrode (200) according to claim 2 or any of claims 3 to 7 when dependent on claim 2, further comprising a multiplexer (260) that is disposed adjacent the connection device (300) and that controls switching of the plurality of conductive, elements (220a-220f).

12. An electrosurgical apparatus (100) for performing electrosurgery on a patient (P), the electrosurgical apparatus comprising:
an electrosurgical generator (120) to produce electrosurgical energy;
**characterised in that** the apparatus comprises
an electrosurgical return electrode (200) according.to any one of the preceding claims that is selectively connectable to the electrosurgical generator (120).

13. The electrosurgical apparatus of claim 12 for performing monopolar surgery, comprising:
an active electrode for supplying the electrosurgical energy to the patient.

14. The electrosurgical apparatus of claim 12 or 13, the electrosurgical generator configured to monitor the temperature of each portion of a patient in contact with the respective conductive element and to disconnect or deactivate an element if the temperature reaches a predetermined level.

15. The electrosurgical apparatus of claim 14, the electrosurgical generator configured to connect or activate an element if the temperature reaches a predetermined level.

## Patentansprüche

1. Elektrochirurgisches Antwortelektrodenfeld (200) zur Verwendung in monopolarer Chirurgie, umfassend:
ein leitendes Feld (210) mit einer Mehrzahl von leitenden Elementen (220a-220f) und mit einer oberen Oberfläche (212) und einer unteren Oberfläche (214), wobei das leitende Feld einen Umriss definiert; **dadurch gekennzeichnet, dass**:
die Antwortelektrode ferner eine Mehrzahl von Temperatursensoren aufweist, wobei jeder Temperatursensor mit einem entsprechenden der Mehrzahl von leitenden Elementen (220a-220f) an der oberen Oberfläche des leitenden Felds wirkverbunden ist, wobei jeder Temperatursensor eingerichtet ist, um eine Temperatur eines Abschnitts eines Patienten (P) in Kontakt mit dem entsprechenden leitenden Element (220a-220f) zu messen.

2. Elektrochirurgische Antwortelektrode (200) nach Anspruch 1, ferner umfassend eine Verbindungsvorrichtung (300), die wahlweise die Übertragung von Hochfrequenzenergie von einer aktiven Elektrode zu zumindest einer der Mehrzahl von leitenden Elementen (220a-220f) ermöglicht, wobei die Verbindungsvorrichtung (300) zumindest eins von verbunden, getrennt, aktiviert, deaktiviert sein kann und an ein leitendes Element angepasst sein kann, wenn die Temperatur eines entsprechenden Abschnitts des Patienten (P) ein vorbestimmtes Niveau erreicht.

3. Elektrochirurgische Antwortelektrode (200) nach einem der vorstehenden Ansprüche, wobei die Mehrzahl von leitenden Elementen (220a-220f) ein Gitter bildet.

4. Elektrochirurgische Antwortelektrode (200) nach einem der vorstehenden Ansprüche, wobei jedes der Mehrzahl von leitenden Elementen (220a-220f) etwa von derselben Größe ist.

5. Elektrochirurgische Antwortelektrode (200) nach einem der Ansprüche 1 bis 3, wobei zumindest eins der Mehrzahl von leitenden Elementen (220a-220f) eine andere Größe als die Verbleibenden der leitenden Elemente aufweist.

6. Elektrochirurgische Antwortelektrode (200) nach Anspruch 5, wobei die leitenden Elemente (220a-220f) entlang des Umrisses des leitenden Feldes relativ kleiner sind als die Verbleibenden der leitenden Elemente.

7. Elektrochirurgische Antwortelektrode (200) nach einem der vorstehenden Ansprüche, ferner umfassend einen Klebeabschnitt (500), der zumindest einen Abschnitt des leitenden Feldes (210) an einen Patienten (P) anklebt.

8. Elektrochirurgische Antwortelektrode (200) nach Anspruch 2 oder einem der Ansprüche 3 bis 7 in Abhängigkeit von Anspruch 2, wobei die Verbindungsvorrichtung (300) Hochfrequenzenergie von einer aktiven Elektrode zu jedem der Mehrzahl der leitenden Elemente (220a-220f) überträgt.

9. Elektrochirurgische Antwortelektrode (200) nach einem der vorstehenden Ansprüche, wobei jeder Temperatursensor (400a-400f) angepasst ist, um einen Patienten (P) zu kontaktieren.

10. Elektrochirurgische Antwortelektrode (200) nach Anspruch 2 oder einem der Ansprüche 3 bis 9 in Abhängigkeit von Anspruch 2, wobei die Verbindungsvorrichtung (300) i) an dem leitenden Feld (210) angeordnet, ii) an einem elektrochirurgischen Generator (120) angeordnet oder iii) zwischen dem leitenden Feld (210) und einem elektrochirurgischen Generator (120) angeordnet ist.

11. Elektrochirurgische Antwortelektrode (200) nach Anspruch 2 oder einem der Ansprüche 3 bis 7 in Abhängigkeit von Anspruch 2, ferner umfassend einen Multiplexer (260), der benachbart zu der Verbindungsvorrichtung (300) angeordnet ist und der ein Schalten der Mehrzahl von leitenden Elementen (220a-220f) steuert.

12. Elektrochirurgische Vorrichtung (100) zum Durchführen von Elektrochirurgie an einem Patienten (P), wobei die elektrochirurgische Vorrichtung umfasst:
einen elektrochirurgischen Generator (120), um elektrochirurgische Energie zu erzeugen;
**dadurch gekennzeichnet, dass** die Vorrichtung eine elektrochirurgische Antwortelektrode (200) nach einem der vorstehenden Ansprüche umfasst, die wahlweise mit dem elektrochirurgischen Generator (120) verbindbar ist.

13. Elektrochirurgische Vorrichtung nach Anspruch 12 zum Durchführen monopolarer Chirurgie, umfassend:
eine aktive Elektrode zum Zuführen der elektrochirurgischen Energie zu dem Patienten.

14. Elektrochirurgische Vorrichtung nach Anspruch 12 oder 13, wobei der elektrochirurgische Generator eingerichtet ist, um die Temperatur von jedem Abschnitt eines Patienten, der in Kontakt mit dem entsprechenden leitenden Element ist, zu überwachen und ein Element zu trennen oder deaktivieren, wenn die Temperatur ein vorbestimmtes Niveau erreicht.

15. Elektrochirurgische Vorrichtung nach Anspruch 14, wobei der elektrochirurgische Generator eingerichtet ist, um ein Element zu verbinden oder zu aktivieren, wenn die Temperatur ein vorbestimmtes Niveau erreicht.

## Revendications

1. Pastille d'électrode de retour électrochirurgicale (200) pour utilisation en chirurgie monopolaire, comprenant:
une pastille conductrice (210) avec une pluralité d'éléments conducteurs (220a, 220f) et ayant une surface supérieure (212) et une surface inférieure (214), la pastille conductrice définissant un périmètre; **caractérisée en ce que**:
l'électrode de retour comprend en outre une pluralité de capteurs de température, chaque capteur de température étant fonctionnellement en prise avec un respectivement de la pluralité d'éléments conducteurs (220a-220f) sur la surface supérieure de la pastille condutrice, où chaque capteur de température est configuré pour mesurer une température d'une portion d'un patient (P) en contact avec l'élément conducteur respectif (220a-220f).

2. Electrode de retour électrochirurgicale (200) selon la revendication 1, comprenant en outre un dispositif de connection (300) qui permet sélectivement le transfert de l'énergie radiofréquence d'une électrode active à au moins un de la pluralité d'éléments conducteurs (220a-220f), où le dispositif de connection (300) peut être au moins un parmi connecté, déconnecté, activé, désactivé et ajusté à un élément conducteur lorsque la température d'une portion correspondante du patient (P) atteint un niveau prédéterminé.

3. Electrode de retour électrochirurgicale (200) selon l'une quelconque des revendications précédentes, où la pluralité d'éléments conducteurs (220a-220f) forme une grille.

4. Electrode de retour électrochirurgicale (200) selon l'une quelconque des revendications précédentes, où chacun de la pluralité des éléments conducteurs (220a-220f) à approximativement la même taille.

5. Electrode de retour électrochirurgicale (200) selon l'une quelconque des revendications 1 à 3, où au moins un de la pluralité des éléments conducteurs (220a, 220f) a une taille différente que les éléments conducteurs restants.

6. Electrode de retour électrochirurgicale (200) selon la revendication 5, où les éléments conducteurs (220a-220f) le long du périmètre de la pastille conductrice sont relativement plus petits que les éléments conducteurs restants.

7. Electrode de retour électrochirurgicale (200) selon l'une quelconque des revendications précédentes, comprenant en outre une portion adhésive (500) qui fait adhérer au moins une portion de la pastille conductrice (210) à un patient (P).

8. Electrode de retour électrochirurgicale (200) selon la revendication 2 ou l'une quelconque des revendications 3 à 7 dépendant de la revendication 2, où le dispositif de connection (300) transfère l'énergie radiofréquence d'une électrode active à chacun de la pluralité d'éléments conducteurs (220a-220f).

9. Electrode de retour électrochirurgicale (200) selon l'une quelconque des revendications précédentes, où chaque capteur de température (400a-400f) est conçu pour venir en contact avec un patient (P).

10. Electrode de retour électrochirurgicale (200) selon la revendication 2 ou l'une quelconque des revendications 3 à 9 dépendant de la revendication 2, où le dispositif de connection (300) est i) disposé sur la pastille conductrice (210), ii) disposé sur un générateur électrochirurgical (120) ou iii) disposé entre la pastille conductrice (210) et un générateur électrochirurgical (120).

11. Electrode de retour électrochirurgicale (200) selon la revendication 2 ou l'une quelconque des revendications 3 à 7 dépendant de la revendication 2, comprenant en outre un multiplexeur (260) qui est disposé d'une manière adjacente au dispositif de connection (300) et qui commande la commutation de la pluralité d'éléments conducteurs (220a-220f).

12. Appareil électrochirurgical (100) pour exécuter une électrochirurgie sur un patient (P), l'appareil électrochirurgical comprenant:
un générateur électrochirurgical (120) pour produire de l'énergie électrochirurgicale;
**caractérisé en ce que** l'appareil comprend
une électrode de retour électrochirurgicale (200) selon l'une quelconque des revendications précédentes qui peut être connectée sélectivement au générateur électrochirurgical (120).

13. Appareil électrochirurgical selon la revendication 12 pour exécuter une chirurgie monopolaire, comprenant:
une électrode active pour fournir l'énergie électrochirurgicale au patient.

14. Appareil électrochirurgical selon la revendication 12 ou 13, le générateur électrochirurgical étant configuré pour surveiller la température de chaque portion d'un patient en contact avec l'élément conducteur respectif et pour déconnecter ou désactiver un élément si la température atteint un niveau prédéterminé.

15. Appareil électrochirurgical selon la revendication 14, le générateur électrochirurgical étant configuré pour être connecté à ou activer un élément si la température atteint un niveau prédéterminé.
